# EUROPÄISCHE PATENTSCHRIFT

(11) **EP 0 584 663 B1**
(45) Veröffentlichungstag und Bekanntmachung des Hinweises auf die Patenterteilung: **25.02.1998**
(21) Anmeldenummer: 93112932.4
(22) Anmeldetag: 12.08.1993
(51) Int. Cl.: C07D 309/08

(54) **Verfahren zur Herstellung von Tetrahydropyran-4-carbonsäure und ihren Estern**
Process for the preparation of tetrahydropyran-4-carboxylic acid and his esters
Procédé de préparation d'acide tétrahydropyranne-4-carboxylique et ses esters

(30) Priorität: 28.08.1992 DE 4228669
(43) Veröffentlichungstag der Anmeldung: 02.03.1994
(73) Patentinhaber: BASF Aktiengesellschaft, 67063 Ludwigshafen (DE)
(72) Erfinder: Borchers, Dirk, Dr., D-8701 Birkenheide (DE); Fischer, Rolf, Dr., D-6900 Heidelberg (DE); Goetz, Norbert, Dr., D-6520 Worms 1 (DE); Kuekenhoehner, Thomas, Dr., D-6737 Boehl-Iggelheim (DE); Schnurr, Werner, Dr., D-6719 Herxheim (DE)

(56) Entgegenhaltungen:
- EP-A- 0 284 969

## Beschreibung

Die vorliegende Erfindung betrifft ein verbessertes Verfahren zur Herstellung von Tetrahydropyran-4-carbonsäure und ihren Estern, bei dem 3-(2'-Hydroxyethyl)-dihydro-2(3H)furanon, seine Ester oder Ether in Gegenwart von sauren Festbettkatalysatoren mit Alkoholen und/oder Wasser umgesetzt werden.

Aus der EP-A-284 969 ist bekannt, Tetrahydropyran-4-carbonsäureester durch Umsetzung von 3-(2'-Hydroxyethyl)-dihydro-2(3H)fura-non oder dessen Estern und Ethern mit Alkoholen in Gegenwart von sauer wirkenden Katalysatoren, herzustellen. Die Umsetzung kann diskontinuierlich oder kontinuierlich als Festbettreaktion mit Festbettkatalysatoren, beispielsweise nach der Sumpf- oder Rieselfahrweise in der Flüssig- oder Gasphase, z.B. im Wirbelbett oder aber mit in der Flüssigphase suspendierten Festbett-Katalysatoren durchgeführt werden. Man kann aber auch in der Flüssigphase homogen gelöste saure Katalysatoren einsetzen.

Die Patentbeispiele zeigen, daß in der Gasphase in Gegenwart von Al203-Wirbelbett-Katalysatoren bei 240 bis 270°C Tetrahydropyrancarbonsäureester-Ausbeuten von nur 36 bis 42 % (bezogen auf eingesetztes 3-(2'-Hydroxyethyl)-dihydro-2(3H)furanon) erzielt werden (Beispiele 1 und 2). Als Nebenprodukte entstehen unter diesen Bedingungen 18 bis 20 % 3-(2'-Methoxyethyl)-dihydro-2(3H)furanon und 16 bis 27 % 3-Vinyldihydro-2(3H)furanon. Beispiel 1b demonstriert, daß sich das Nebenprodukt 3-(2'-Methoxyethyl)-di-hydro-2(3H)furanon mit Methanol in 26 %iger Ausbeute zu Tetrahydropyran-4-carbonsäuremethylester umsetzen läßt. Dagegen gelingt die Umsetzung des Nebenprodukts 3-Vinyldihydro-2(3H)furanon mit Methanol nach Beispiel 1c nur mit 10 %iger Ausbeute.

Der vorliegenden Erfindung lag daher die Aufgabe zugrunde, den zuvor genannten Nachteilen abzuhelfen und die Tetrahydropyrancarbonsäureester-Ausbeute zu steigern.

Demgemäß wurde ein neues und verbessertes Verfahren zur Herstellung von Tetrahydropyran-4-carbonsäure und ihren Estern der allgemeinen Formel I 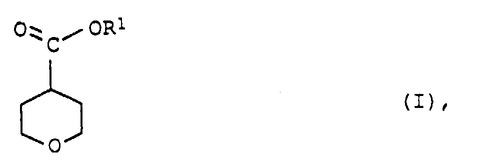 in der
- R¹: Wasserstoff, C₁- bis C₆-Alkyl, C₅- bis C₈-Cycloalkyl oder Aryl
bedeutet, durch Umsetzung von Dihydro-2(3H)furanonen der allgemeinen Formel II 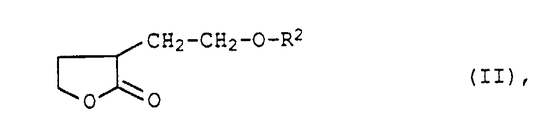 in der
- R²: Wasserstoff, C₁- bis C₆-Alkyl oder -CO-R³ und
- R³: Wasserstoff oder C₁- bis C₆-Alkyl
bedeuten, mit Wasser oder Alkoholen der allgemeinen Formel III

R¹-OH (III),

in der R¹ die oben genannte Bedeutung hat, bei Temperaturen von 200 bis 350°C in Gegenwart von sauren Heterogenkatalysatoren gefunden, welches dadurch gekennzeichnet ist, daß man fest angeordnete saure Heterogenkatalysatoren einsetzt.

Das erfindungsgemäße Verfahren läßt sich wie folgt durchführen:

Die Umsetzung von Dihydro-2(3H)furanonen II mit Alkoholen III kann in der Gasphase oder in der Flüssigphase an fest angeordneten sauren Heterogenkatalysatoren bei Temperaturen von 200 bis 350°C, bevorzugt 200 bis 300°C, besonders bevorzugt 230 bis 270°C und Drücken von von 0,01 bis 100 bar, bevorzugt 0,1 bis 5 bar, besonders bevorzugt 0,5 bis 2 bar, insbesondere bei Normaldruck (Atmosphärendruck) durchgeführt werden. Man arbeitet im allgemeinen mit Katalysatorbelastungen von 0,1 bis 10, insbesondere von 0,1 bis 5 g Dihydro-2(3H)furanon II pro g Katalysator und Stunde.

Das Molverhältnis von Alkohol III zum Dihydro-2(3H)furanon II beträgt vorteilhaft 0,5 : 1 bis 10 : 1, insbesondere 1 : 1 bis 5 : 1.

Es kann zweckmäßig sein, dem Gemisch aus Dihydro-2(3H)furanon II und Alkohol III Wasser zuzusetzen. Die Wassermenge kann 0,01 bis 5 Mol, insbesondere 0,5 bis 2 Mol Wasser pro Mol Dihydro-2(3H)furanon II betragen.

Das Gemisch aus Dihydro-2(3H)furanon, Alkohol und gegebenenfalls Wasser kann in einem separaten Verdampfer vorverdampft und dann auf das Katalysatorbett gefahren werden. Es ist aber auch möglich, das Gemisch im Reaktor selbst zu verdampfen, z.B. in einer auf die notwendige Temperatur geheizten Schicht aus inerten Füllkörpern, die dem Katalysatorbett vorgelagert ist.

Die Herstellung von Tetrahydropyran-4-carbonsäureestern I kann z.B. so erfolgen, daß man ein Gemisch aus dem Dihydro-2(3H)furanon II und dem jeweiligen Alkohol III verdampft und dann, gegebenenfalls zusammen mit einem Inertgas wie Stickstoff, Kohlendioxid oder Argon, bei der gewünschten Reaktionstemperatur über einen fest angeordneten Katalysator leitet. Der Reaktionsaustrag kann mittels geeigneter Kühlvorrichtung kondensiert und anschließend durch fraktionierende Destillation aufgearbeitet werden. Nicht umgesetztes 3-(2'-Hydroxyethyl)-dihydro-2(3H)furanon bzw. dessen Ether oder Ester können in die Reaktion zugeführt werden.

Als fest angeordnete Heterogenkatalysatoren eignen sich Festbettkatalysatoren, z.B. sauer wirkende Oxide von Elementen der III. und IV. Hauptgruppe sowie der IV. bis VI. Nebengruppe des Periodensystems der Elemente. Beispielsweise seien heterogene saure Katalysatoren genannt, wie Siliciumdioxid in Form von Kieselgel, Kieselgur, Quarz, weiterhin Titandioxid, Zirkondioxid, Phosphorpentoxid, Vanadiumpentoxid, Bortrioxid, Aluminiumoxid, Chromoxide, Molybdänoxide, Wolframoxide oder deren Gemische. Es sind auch Zeolithe, wie z.B. Y-Zeolithe geeignet. Als Katalysator besonders bevorzugt ist Aluminiumoxid.

3-(2'-Hydroxyethyl)-dihydro-2(3H)furanon kann z.B. durch Umsetzung von Acetessigester mit Ethylenoxid hergestellt werden (EP-A-246 581).

Die Substituenten R¹, R² und R³ in den Verbindungen I, II und III haben folgende Bedeutungen: R¹, R² und R³
- unabhängig voneinander
- Wasserstoff,
- C₁- bis C₆-Alkyl, bevorzugt C₁- bis C₄-Alkyl wie Methyl, Ethyl, n-Propyl, iso-Propyl, n-Butyl, iso-Butyl, sec.-Butyl und tert.-Butyl, besonders bevorzugt Methyl und Ethyl,
_{R}1
- zusätzlich
- C₅- bis C₈-Cycloalkyl wie Cyclopentyl, Cyclohexyl, Cycloheptyl und Cyclooctyl, bevorzugt Cyclopentyl, Cyclohexyl und Cyclooctyl, besonders bevorzugt Cyclopentyl und Cyclohexyl
- Aryl wie Phenyl, 1-Naphthyl, 2-Naphthyl, 1-Anthryl, 2-Anthryl und 9-Anthryl, bevorzugt Phenyl, 1-Naphthyl und 2-Naphthyl, besonders bevorzugt Phenyl,
R²
- zusätzlich
- -CO-R³ .

Geeignete Dihydro-2-(3H)furanone II sind z.B. 3-(2'-Hydroxy-ethyl)-dihydro-2(3H)furanon, dessen Hydroxyethylgruppe z.B. zum Methyl-, Ethyl-, Propyl-, Butylether verethert oder z.B. mit Ameisensäure, Essigsäure, Propionsäure, Buttersäure-, n- und i-Valeriansäure, Benzoesäure verestert sein kann. Besonders bevorzugte Ausgangsverbindungen II sind die Ester des 3-(2'-Hydroxy-ethyl)-dihydro-2(3H)furanons mit Essigsäure und Propionsäure. Geeignete Alkohole der Formel III sind beispielsweise Methanol, Ethanol, n-Propanol, i-Propanol, tert.-Butanol, n-Butanol, 1-Butanol, sec.-Butanol, n-Pentanol, n-Hexanol, Phenol, Cyclopentanol, Cyclohexanol. Besonders geeignet sind Methanol, Ethanol und Propanole.

Durch das erfindungsgemäße Verfahren läßt sich die Ausbeute an Tetrahydropyran-4-carbonester I gegenüber EP-A-284.969 um rund 20 % steigern. Außerdem wird die Menge an 5-Oxaspiro[2.4]heptan-4-on, 3-Ethylidendihydro-2(3H)furanon und 3-Vinyldihydro-2(3H)furanon zurückgedrängt.

Es war überraschend, daß sich durch den Wechsel von Wirbelbettzu Festbettfahrweise sowohl die Tetrahydropyran-4-carbonsäureester-Ausbeute als auch das Verhältnis von 3-(2'-Alkoxyethyl)-dihydro-2(3H)furanon zu 5-Oxaspiro [2.4]heptan-4-on/3-Vinyldihydro-2(3H)furanon beträchtlich steigern läßt.

Die nach dem erfindungsgemäßen Verfahren erhältlichen Tetrahydropyran-4-carbonsäureester I stellen wertvolle Zwischenprodukte dar, die z.B. zu dem gesuchten Tetrahydropyran-4-carbaldehyd weiterverarbeitet werden können (DE-A-31 21 355, DE-A-33 14 816, DE-A-33 40 265, DE-A-38 21 197 und DE-A-40 39 918).

### Beispiele

### Beispiel 1

Pro Stunde wurden 170 g einer Lösung, die aus 57 Gew.-% 3-(2'-Acetoxyethyl)-dihydro-2(3H)furanon und 43 Gew.-% Methanol bestand, in einen Verdampfer (300°C) gepumpt und von dort mit 80 l/h Stickstoff bei einer Reaktortemperatur von 250°C über 560 g (950 ml) Al₂O₃ (1,3-mm-Stränge) in Rieselfahrweise geleitet. Der gasförmige Reaktionsaustrag wurde in Kühlfallen kondensiert und gaschromatographisch analysiert. Dabei wurden 61 Mol.-% Tetrahydropyran-4-carbonsäuremethylester, 26 Mol.-% 3-(2'-Methoxy-ethyl) -dihydro-2(3H)furanon und 5 Mol.-% 5-Oxaspiro[2.4]heptan-4-on gebildet. Der 3-(2'-Acetoxyethyl)-dihydro-2(3H)furanon-Umsatz betrug 97 %.

### Beispiel 2

Pro Stunde wurden 25 g einer Lösung, die aus 53 Gew.-% 3-(2'-Acetoxyethyl)-dihydro-2(3H)furanon, 39 Gew.-% Methanol und 8 Gew.-% Wasser bestand, verdampft und in einem Rohrreaktor bei 250°C über 90 g Aluminiumoxid-Katalysator (d = 1,5 mm) geleitet. Der gasförmige Reaktionsaustrag wurde in Kühlfallen kondensiert und gaschromatographisch analysiert. Die Versuchsdauer betrug 12 Stunden, der Gesamteinsatz 600 g. Dabei wurden 62 Mol.-% Tetrahydropyran-4-carbonsäuremethylester, 20 Mol.-% 3-(2'-Methoxy-ethyl)-dihydro-2 (3H)furanon und 3 Mol.-% 5-Oxaspiro[2.4]heptan-4-on gebildet. Der Umsatz an 3-(2'-Acetoxyethyl)-dihydro-2(3H)furanon war > 99 %.

### Beispiel 3

Pro Stunde wurden 25 g einer Lösung, die aus 45 Gew.-% 3-(2'-Hy-droxyethyl)-dihydro-2(3H)furanon, 45 Gew.-% Methanol und 10 Gew.-% Wasser bestand, verdampft und in einem Rohrreaktor bei 250°C über 90 g Aluminiumoxid-Katalysator (d = 1,5 mm) geleitet. Der gasförmige Reaktionsaustrag wurde in Kühlfallen kondensiert und gaschromatographisch analysiert. Die Versuchsdauer betrug 60 Stunden, der Gesamteinsatz 1500 g. Dabei wurden 57 Mol.-% Tetrahydropyran-4-carbonsäuremethylester, 32 Mol.-% 3-(2'-Methoxyethyl)-dihydro-2 (3H)furanon und 4 Mol.-% 5-Oxaspiro [2.4]heptan-2-on gebildet, der Umsatz an 3-(2'-Hydroxyethyl)-dihydro-2(3H)furanon betrug 97 %.

### Beispiel 4

Während einer Versuchsdauer von 36 Stunden wurden insgesamt 900 g einer Lösung, die aus 53 Gew.-% 3-(2'-Methoxyethyl)-dihydro-2(3H)furanon und 47 Gew.-% Methanol bestand, verdampft und bei 2500C in einem Rohrreaktor über 90 g Aluminiumoxid-Katalysator (d = 1,5 mm) geleitet. Nach Kondensation des gasförmigen Reaktionsaustrages wurde eine gaschromatographische Analyse vorgenommen. Der Umsatz an 3-(2'-Methoxyethyl)-dihydro-2(3H)furanon betrug 53 %. Es wurden 46 Mol.-% Tetrahydropyran-4-carbonsäuremethylester und 7 Mol.-% 5-Oxaspiro[2.4]heptan-2-on gebildet.

### Beispiel 5

In ein zylindrisches Reaktionsrohr mit einem Volumen von 250 ml wurden Al₂O₃-Stränge (1,5 mm Durchmesser, 3 mm Länge) eingefüllt. Bei einer Reaktionstemperatur von 250°C wurden nun stündlich 20 g einer Mischung, die aus 65 Gew.-% 3-(2'-Hydroxyethyl)-dihydro-2(3H)furanon und 35 Gew.-% Wasser bestand, über diesen Katalysator geführt. Gleichzeitig wurden 200 1 Stickstoff bei Normaldruck im Gleichstrom durch das Reaktionsrohr geleitet. Die Versuchsdauer betrug 8 Stunden, wobei insgesamt 158 g Reaktionsprodukt anfielen. Durch Andestillation wurde das im Überschuß vorhandene Wasser entfernt. Das danach isolierte Rohprodukt enthielt
- 28,0 Gew.-%: 3-(2'-Hydroxyethyl)-dihydro-2(3H)furanon (Ausgangsprodukt)
- 17,4 Gew.-%: Tetrahydropyran-4-carbonsäure
- 29,1 Gew.-%: Tetrahydropyran-4-carbonsäure- [2-(dihydro-2(3H)furanon-3-yl)-ethyl]-ester

Bei einem Umsatz von 72 % ergibt sich eine Selektivität für Tetrahydropyran-4-carbonsäure von 24 %, für den Ester eine Selektivität von 40 %.

### Beispiel 6

In ein zylindrisches Reaktionsrohr mit einem Volumen von 250 ml wurden Al₂O₃-Stränge (1,5 mm Durchmesser, 3 mm Länge) eingefüllt. Bei einer Reaktionstemperatur von 250°C und einem Druck von 500 mbar wurden stündlich 30 g einer Mischung aus 57 Gew.-% 3-(2'-Acetoxyethyl)-dihydro-2(3H)furanon und 43 Gew.-% Methanol über diesen Katalysator geführt. Das gasförmige Reaktionsprodukt wurde in Kühlfallen kondensiert und seine Zusammensetzung gaschromatographisch analysiert. Unter diesen Reaktionsbedingungen wurden 59 mol-% Tetrahydropyran-4-carbonsäuremethylester, 18 mol-% 3-(2'-Methoxyethyl)-dihydro-2(3H)furanon und 5 mol-% 5-Oxaspiro [2.4]heptan-2-on gebildet. Der 3-(2'-Acetoxyethyl)-dihydro-2(3H)furanon-Umsatz betrug über 95 %.

### Beispiel 7

In einem Rohrreaktor wurden pro Stunde 38 g einer Lösung, die aus 37 Gew.-% 3-(2'-Acetoxyethyl)-dihydro-2(3H)furanon, 19 Gew.% 3-(2'-Methoxyethyl)dihydro-2(3H)furanon und 44 Gew.% Methanol bestand, unter Zusatz von 10 l/h Stickstoff bei 250°C über 140 g Aluminiumoxid-Katalysator (1,5 mm Stränge) geleitet. Der gasförmige Reaktionsaustrag wurde in Kühlfallen kondensiert und gaschromatographisch analysiert. Dabei wurden 53 mol-% Tetrahydropyran-4-carbonsäuremethylester, 41 mol-% 3-(2'-Methoxy-ethyl)-dihydro-2(3H)furanon und 3 mol-% 5-Oxaspiro[2,4]heptan-2-on gebildet. Der Umsatz an 3-(2'-Acetoxyethyl)-dihydro-2(3H)furanon betrug über 99 %.

## Patentansprüche

1. Verfahren zur Herstellung von Tetrahydropyran-4-carbonsäure und ihren Estern der allgemeinen Formel I 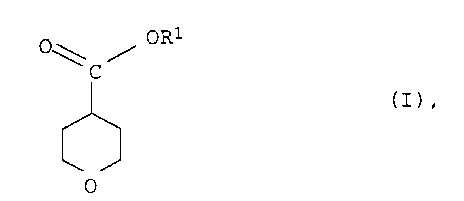 in der
R¹ Wasserstoff, C₁- bis C₆-Alkyl, C₅- bis C₈-Cycloalkyl oder Aryl
bedeutet, durch Umsetzung von Dihydro-2(3H)furanonen der allgemeinen Formel II 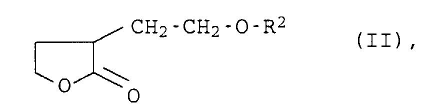 in der
R² Wasserstoff, C₁- bis C₆-Alkyl oder -CO-R³ und
R³ Wasserstoff oder C₁- bis C₆-Alkyl
bedeuten, mit Wasser oder Alkoholen der allgemeinen Formel III
R¹-OH (III),
in der R¹ die oben genannte Bedeutung hat, bei Temperaturen von 200 bis 350°C in Gegenwart von sauren Heterogenkatalysatoren, dadurch gekennzeichnet, daß man fest angeordnete saure Heterogenkatalysatoren einsetzt und die Umsetzung in Festbettfahrweise durchführt.

2. Verfahren zur Herstellung von Tetrahydropyran-4-carbonsäure und ihren Estern nach Anspruch 1, dadurch gekennzeichnet, daß man die Umsetzung bei Temperaturen von 200 bis 300°C vornimmt.

3. Verfahren zur Herstellung von Tetrahydropyran-4-carbonsäure und ihren Estern nach Anspruch 1, dadurch gekennzeichnet, daß man die Umsetzung bei Temperaturen von 230 bis 270°C vornimmt.

4. Verfahren zur Herstellung von Tetrahydropyran-4-carbonsäure und ihren Estern nach Anspruch 1, dadurch gekennzeichnet, daß man als saure Heterogenkatalysatoren sauer wirkende Oxide von Elementen der III. und/oder IV. Hauptgruppe und/oder der IV. bis VI. Nebengruppe einsetzt.

5. Verfahren zur Herstellung von Tetrahydropyran-4-carbonsäure und ihren Estern nach Anspruch 1 und 3, dadurch gekennzeichnet, daß man als sauren Heterogenkatalysator Aluminiumoxid einsetzt.

6. Verfahren zur Herstellung von Tetrahydropyran-4-carbonsäure und ihren Estern nach Anspruch 1, dadurch gekennzeichnet, daß man als Butyrolacton II 3-(2'-Acetoxyethyl)-dihydro-2(3H)-furanonund/oder3-(2'-Hydroxyethyl)-dihydro-2(3H)furanon und/oder 3- (2'-Methoxyethyl)-dihydro-2 (3H)furanon einsetzt.

## Claims

1. A process for preparing tetrahydropyran-4-carboxylic acid and its esters of the formula I 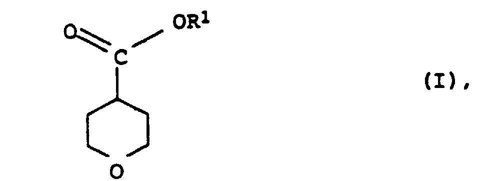 where
R¹ is hydrogen, C₁-C₆-alkyl, C₅-C₈-cycloalkyl or aryl, by reacting dihydro-2 (3H) furanones of the formula II 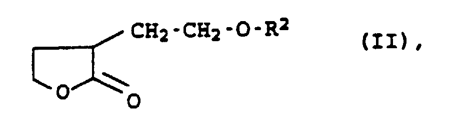 where
R² is hydrogen, C₁-C₆-alkyl or -CO-R³ and
R³ is hydrogen or C₁-C₆-alkyl,
with water or alcohols of the formula III
R¹-OH (III),
where R¹ is as defined above, at from 200 to 350°C in the presence of acid heterogeneous catalysts, wherein fixed-bed acid heterogeneous catalysts are used and the reaction is carried out in the fixed-bed mode.

2. A process for preparing tetrahydropyran-4-carboxylic acid and its esters as claimed in claim 1, wherein the reaction is carried out at from 200 to 300°C.

3. A process for preparing tetrahydropyran-4-carboxylic acid and its esters as claimed in claim 1, wherein the reaction is carried out at from 230 to 270°C.

4. A process for preparing tetrahydropyran-4-carboxylic acid and its esters as claimed in claim 1, wherein the acid heterogeneous catalysts used are acidic oxides of elements of main groups III and/or IV and/or transition groups IV to VI.

5. A process for preparing tetrahydropyran-4-carboxylic acid and its esters as claimed in claim 1 or 3, wherein the acid heterogenous catalyst used is aluminium oxide.

6. A process for preparing tetrahydropyran-4-carboxylic acid and its esters as claimed in claim 1, wherein the butyrolactone II used is 3-(2'-acetoxyethyl)dihydro-2(3H)-furanone and/or 3- (2'-hydroxyethyl)dihydro-2(3H)-furanone and/or 3- (2'-methoxyethyl)dihydro-2(3H)-furanone.

## Revendications

1. Procédé pour préparer l'acide tétrahydropyranne-4-carboxylique et ses esters, répondant à la formule générale I 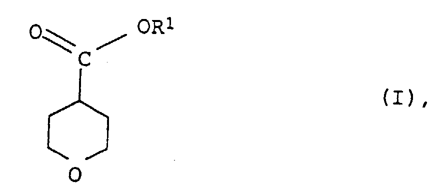 dans laquelle
R¹ représente l'hydrogène, un groupe alkyle en C1-C6, cycloalkyle en C5-C8 ou aryle,
par réaction de dihydro-2(3H)furannones de formule générale II 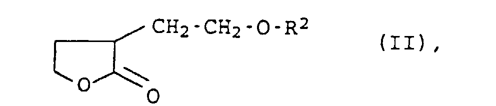 dans laquelle
R² représente l'hydrogène, un groupe alkyle en C1-C6 ou -CO-R³ et
R³ représente l'hydrogène ou un groupe alkyle en C1-C6,
avec l'eau ou des alcools de formule générale III
R¹-OH (III)
dans laquelle R¹ a les significations indiquées ci-dessus, à des températures de 200 à 350°C, en présence de catalyseurs hétérogènes acides, caractérisé par le fait que l'on utilise des catalyseurs hétérogènes acides en disposition fixe et que l'on exécute la réaction dans une opération en lit fixe.

2. Procédé pour préparer l'acide tétrahydropyranne-4-carboxylique et ses esters selon la revendication 1, caractérisé par le fait que l'on effectue la réaction à des températures de 200 à 300°C.

3. Procédé pour préparer l'acide tétrahydropyranne-4-carboxylique et ses esters selon la revendication 1, caractérisé par le fait que l'on effectue la réaction à des températures de 230 à 270°C.

4. Procédé pour préparer l'acide tétrahydropyranne-4-carboxylique et ses esters selon la revendication 1, caractérisé par le fait que l'on utilise, en tant que catalyseurs hétérogènes acides, des oxydes acides des éléments des groupes principaux III et/ou IV et/ou des sous-groupes IV à VI.

5. Procédé pour préparer l'acide tétrahydropyranne-4-carboxylique et ses esters selon les revendications 1 et 3, caractérisé par le fait que l'on utilise, en tant que catalyseur hétérogène acide, une alumine.

6. Procédé pour préparer l'acide tétrahydropyranne-4-carboxylique et ses esters selon la revendication 1, caractérisé par le fait que l'on utilise, en tant que butyrolactone II, la 3-(2'-acétoxyéthyl)-dihydro-2(3H)furannone et/ou la 3-(2'-hydroxyéthyl)-dihydro-2(3H)furannone et/ou la 3-(2'-méthoxyéthyl)-dihydro-2(3H)furannone.
